# EUROPÄISCHE PATENTSCHRIFT

(11) **EP 0 305 459 B1**
(45) Veröffentlichungstag und Bekanntmachung des Hinweises auf die Patenterteilung: **07.01.1993**
(21) Anmeldenummer: 88902390.9
(22) Anmeldetag: 15.03.1988
(51) Int. Cl.: A61B 5/14

(54) **BLUTENTNAHMEVORRICHTUNG**
DEVICE FOR TAKING BLOOD
DISPOSITIF DE PRELEVEMENT DE SANG

(30) Priorität: 17.03.1987 DE 8703971 U
(43) Veröffentlichungstag der Anmeldung: 08.03.1989
(73) Patentinhaber: Ballies, Uwe Werner, Dr., 24103 Kiel (DE)
(72) Erfinder: Ballies, Uwe Werner, Dr., 24103 Kiel (DE)
(74) Vertreter: Tönnies, Jan G., Dipl.-Ing.
(86) Internationale Anmeldenummer: DE8800147
(87) Internationale Veröffentlichungsnummer: WO8806861

(56) Entgegenhaltungen:
- DE-A- 2 451 398
- US-A- 3 706 306
- US-A- 3 914 985
- US-A- 3 931 010
- US-A- 4 459 997

## Beschreibung

Die Erfindung betrifft eine Blutentnahmevorrichtung mit einem aus einem nachgiebigen Kunststoff gefertigten ersten Röhrchen.

Blutentnahmeröhrchen sind in vielfältiger Ausgestaltung bekannt. Sie dienen dazu, das Blut zur Untersuchung aufzunehmen.

Aus der DE-OS 27 11 336 ist eine Blutentnahmevorrichtung bekannt, bei der ein aus einem elastischen Kunststoff gefertigtes Röhrchen einen Trennkörper aufnimmt, dessen spezifisches Gewicht zwischen denen der zu trennenden Komponenten des Blutes liegt und der sich bei Zentrifugieren zwischen die beiden zu trennenden Komponenten anordnet und nach Beendigung des Zentrifugierens diese durch Absperren des Querschnitts des Trennröhrchens zuverlässig voneinander trennt. Aus der DE-PS 31 01 733 ist es weiter bekannt, das Trennelement asymmetrisch auszubilden.

Die bekannten Trennröhrchen beruhen auf der Erkenntnis, daß der bei dem Zentrifugieren entstehende hohe Druck der Flüssigkeitssäule zu einer Weitung des Materials des Trennröhrchens führt, wodurch der Trennkörper seine Dichtwirkung verliert und eine Trennung der Komponenten des Blutes durch den Trennkörper ermöglicht wird. Voraussetzung ist damit eine gewisse Elastizität des Materials des Trennröhrchens.

Es ist jedoch für bestimmte Anwendungsfälle wünschenswert, daß die Blutentnahmevorrichtung eine größere Festigkeit und damit eine größere Widerstandsfähigkeit gegenüber mechanischen Einwirkungen aufweist. Eine Verwendung eines festeren Materials ist weiter dann vorteilhaft, wenn die Röhrchen mit einem Unterdruck geliefert werden, der über eine längere Zeit aufrechterhalten bleiben soll.

Der Erfindung liegt damit die Aufgabe zugrunde, eine Blutnahmevorrichtung zu schaffen, die gegenüber mechanischen Einwirkungen eine hohe Widerstandsfähigkeit hat. Dabei soll die Blutentnahmevorrichtung auch in Verbindung mit einem sich bei dem Zentrifugieren zwischen die zu trennenden Komponente legenden Trennkörper verwendbar sein.

Erfindungsgemäß wird diese Aufgabe durch die im kennzeichnenden Teil des Anspruch 1 angegebenen Merkmale gelöst. Die Unteransprüche geben vorteilhafte Ausbildungen der Erfindung an.

Weitere Merkmale und Vorteile der Erfindung ergeben sich aus der Beschreibung, in der Ausführungsbeispiele der Erfindung anhand einer Zeichnung erläutert wird. Dabei zeigt:
- Fig. 1: eine Schnittdarstellung eines ersten Ausführungsbeispiels, und
- Fig. 2: eine Schnittdarstellung eines zweiten Ausführungsbeispiels, und
- Fig. 3: eine Schnittdarstellung eines dritten Ausführungsbeispiels.

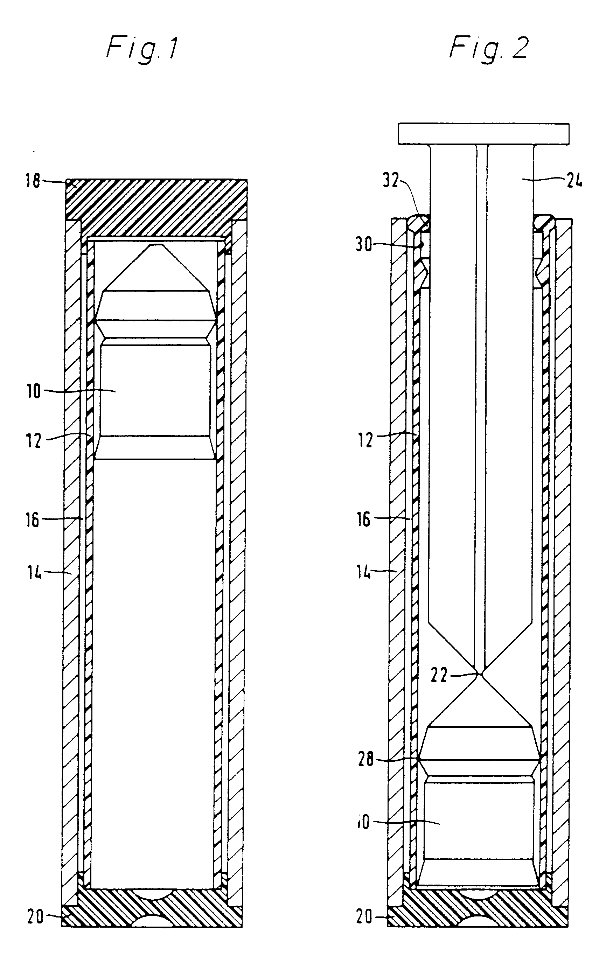

Die in Fig. 1 gezeigte Blutentnahmevorrichtung besteht aus einem ersten, inneren Röhrchen 12 und einem zweiten, äußeren Röhrchen 14. Beide Röhrchen sind beidseitig von Stopfen 18, 20 verschlossen, die das innere Röhrchen 12 zugleich in dem äußeren Röhrchen 14 zentrieren. Der Außendurchmesser des inneren Röhrchens 12 ist geringer als der Innendurchmesser des Röhrchens 14, so daß sich ein Ringspalt 16 zwischen den Wandungen der beiden Röhrchen 12, 14 ausbildet.

Das innere Röhrchen 12 und der Ringspalt 16 sind evakuiert. Bei Durchstechen des Stopfens 20 mittels einer Kanüle wird daher das von der Blutentnahmevorrichtung aufzunehmende Blut automatisch in das innere Röhrchen 12 eingesogen. Nach Aufnahme des Blutes kann die Blutentnahmevorrichtung, die wegen der Doppelschaligkeit und dem Schutz der Außenkanten des äußeren Röhrchens durch die vorzugsweise aus Gummi gefertigten Stopfen 18, 20 besonders gesichert ist, zur Analyse verschickt werden. Bei der vor der eigentlichen Analyse stattfindenden Zentrifugation zur Trennung der Bestandteile des Blutes wandert das Trennelement 10 in an sich aus der DE-OS 27 11 336, der DE-PS 27 34 720 und der DE-PS 31 01 733 bekannten Weise zwischen die zu trennenden Komponenten des Blutes und sperrt hier. Um dies zu ermöglichen ist der Ringspalt 16 zwischen den beiden Röhrchen 12, 14 derart zu wählen, daß eine Weitung des inneren Röhrchens 12 bei dem Zentrifugieren möglich bleibt (das äußere Röhrchen 14 ist aus einem Material gefertigt, das sich bei der Zentrifugation im wesentlichen nicht weitet.)

Der Stopfen 18, der weder bei dem Einfüllen des Blutes in das innere Röhrchen 12, noch bei dem Zentrifugieren von dem aufgenommenen Blut oder einer Komponente des Blutes benetzt worden ist, wird nach dem Zentrifugieren entfernt, bevor das Röhrchen in den Analysator eingesetzt wird. Wesentlich ist dabei, daß weder der Stopfen 18 noch der nach dem Entfernen des Stopfens 18 offen liegende Innenbereich des inneren Röhrchens 12 mit infektiösem Material in Berührung kommt. Die Blutentnahmevorrichtung nach diesem Ausführungsbeispiel ist damit besonders sicher.

Die Doppelschaligkeit wirkt einem Austreten von Blut bei Beschädigung durch mechanische Einwirkung entgegen.

Das Blutentnahmeröhrchen öffnet sich bei dem Zentrifugieren automatisch, da der Trennkörper ja bei dem Zentrifugieren eine Position zwischen den voneinander zu trennenden Komponenten des Blutes annimmt und die obere Öffnung des Röhrchens freigibt. Dies ist besonders vorteilhaft, weil ein Arbeitsgang erspart wird. Weiter ist es in Hinblick auf die Kontaminationsgefahr vorteilhaft, das ein manuelles Öffnen nicht erforderlich ist.

Von erheblicher Bedeutung ist bei diesem Ausführungsbeispiel, daß auch der Ringspalt 16 evakuiert wird: Das aus einem nachgiebigen Material gefertigte innere Röhrchen 12 ist nicht ausreichend dicht, so daß sich das Vakuum schon relativ bald abbauen würde. Weiter würde das Vakuum in dem Innenröhrchen 12 ein Einfallen des inneren Röhrchens 12 bewirken. Sowohl dem Abbau des Unterdrucks in dem Röhrchen 12 als auch dem Einfallen des Röhrchens 12 wirkt eine Evakuierung des Ringspaltes 16 entgegen. Das Material des äußeren Röhrchens 14 wiederum ist ausreichend fest, um über längere Zeit einem Abbau des Unterdrucks entgegenzuwirken. Das äußere Röhrchen kann bei diesem Ausführungsbeispiel insbesondere auch aus Glas gefertigt sein. Der Ringspalt 16, der auch nach Füllung des inneren Röhrchens 12 evakuiert ist, hat weiter eine thermisch isolierende Funktion.

Der Ringspalt 16 ermöglicht es weiter, die Blutentnahmevorrichtung nach dem Zentrifugieren tiefzukühlen und so zu lagern oder zu versenden (dies ist bei den vorbekannten Systemen mit einer starren Wandung nicht möglich, weil die Ausdehnung der Wasseranteile des Blutes bei dem Einfrieren die Wandungen sprengen würde). Das System bleibt bei dem Zentrifugieren beidseitig geschlossen und kann auch versiegelt zentrifuguiert werden.

Bei dem in Fig. 2 dargestellten Ausführungsbeispiel ist lediglich das eine Ende der Röhrchen 12, 14 mit einem Stopfen 20 verschlossen. Das innere Röhrchen 12 ist wiederum mit einem Trennelement 10 versehen, wobei dieses Trennelement 10 über eine Sollbruchstelle 22 mit einer Kolbenstange 24 verbunden ist, wie dies im einzelnen in der DE-PS 27 34 720 beschrieben ist. Weiter weist das innere Röhrchen an seinem anderen Ende einen einen Sperring 32 bildende Verdickung der Innenwandung auf. Dieser Sperring 32 verhindert ein versehentliches Herausziehens des Trennelements 10 aus dem inneren Röhrchen 12. Weiter ist unterhalb des Sperrings 32 eine Einrastnut 30 vorgesehen, die das Trennelement in einer herausgezogenen Stellung gegen den in dem Röhrchen gebildeten Unterdruck hält. Dabei ist die Einrastnut 30 derart auszubilden, daß die bei dem Zentrifugieren auftretenden Kräfte ein Lösen des Trennelements aus der Einrastnut 30 bewirken.

In der Anwendung unterscheidet sich das Ausführungsbeispiel nach Fig. 2 also von dem nach Fig. 1 dadurch, daß in dem Röhrchen 12 kein Vakuum vorgelegt ist. Es kann jedoch unmittelbar vor dem Durchstechen des Stopfens 20 mittels der Kanüle ein solches Vakuum gezogen werden, indem die Kolbenstange 24 nach oben aus dem Röhrchen herausgezogen wird. Dabei verhindert das äußere Röhrchen 14 das Einfallen des aus einem weichen Material gefertigten inneren Röhrchens 12. Nach Herausziehen der Kolbenstange aus dem Röhrchen 12 wird dieses an der Sollbruchstelle 22 abgebrochen. Das Trennelement 10 bildet sodann den das innere Röhrchen 12 verschließenden Stopfen. Auch bei dem zweiten Ausführungsbeispiel wird das innere Röhrchen bei dem Zentrifugieren automatisch geöffnet.

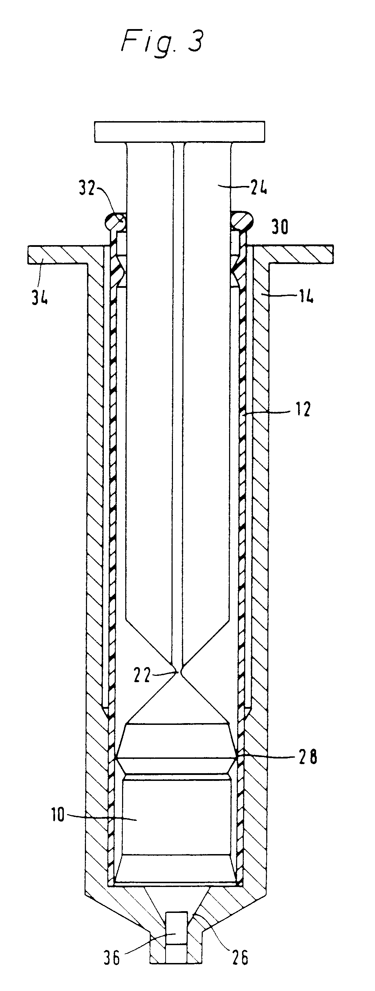

Bei dem in Fig. 3 gezeigten Auführungsbeispiel ist das äußere Röhrchen 14 mit einem Kanülenstutzen 26 versehen, der zur Aufnahme einer Kanüle dient. Dabei kann ein Ventil 36, das ein Rückströmen des Blutes verhindert, vorgesehen sein. Wenn das Blut durch einen zuvor in dem inneren Röhrchen aufgebauten Unterdruck eingesogen werden soll, ist der Kanülenstutzen 26 mittels eines besonderen, von der Kanüle zu durchstechenden Stopfen zu versehen.

Bei dem in Fig. 3 gezeigten Ausführungsbeispiel ist die Länge des äußeren Röhrchens geringer als die Länge des inneren Röhrchens. Weiter ist das äußere Röhrchen hier an seinem oberen Ende mit einer Griffplatte 34 versehen.

Weiter ist bei dem dritten Ausführungsbeispiel dargestellt, daß in dem unteren Bereich der Blutentnahmevorrichtung der Innendurchmesser der Wandung des äußeren Röhrchens 14 dem Außendurchmesser der Wandung des inneren Röhrchens 12 entsprechend ausgebildet sein kann, da bei der Zentrifugation das Trennelement diesen Bereich nicht erreicht. Bei einer derartigen Ausbildung mit einem Passitz zwischen dem inneren Röhrchen 12 und dem äußeren Röhrchen 14 wird eine höhere mechanische Festigkeit der Gesamtanordnung erreicht.

## Patentansprüche

1. Blutentnahmevorrichtung mit einem aus nachgiebigen Material gefertigten ersten Röhrchen (12), wobei das Röhrchen (12) ein Trennelement (10) aufnimmt, des sich bei Zentrifugieren aufweitet und so eine axiale Verschiebung des ansonsten dichtend an der Röhrchenwandung anliegenden Trennelementes (10) ermöglicht,
gekennzeichnet durch ein zweites, aus einem nicht wesentlich nachgiebigen Material gefertigten Röhrchen (14), welches das erste Röhrchen (12) unter Ausbildung eines Ringspaltes (16) zwischen den Wandungen beider Röhrchen (12, 14) umgibt, und Mitteln zum Verschließen des wenigstens einen Endes des ersten Röhrchens.

2. Blutentnahmevorrichtung nach Anspruch 1, dadurch gekennzeichnet, daß beide Röhrchen (12, 14) an wenigstens einem der beiden Enden mittels eines Stopfens (18) verschlossen sind, wobei der eine Stopfen (18) von einer Kanüle durchdringbar ist.

3. Blutentnahmevorrichtung nach Anspruch 2, dadurch gekennzeichnet, daß beide Röhrchen (12, 14) an beiden Enden mittels Stopfen (18, 20) verschlossen sind.

4. Blutentnahmevorrichtung nach Anspruch 2 oder 3, dadurch gekennzeichnet, daß der eine Stopfen (18) von einer Kanüle durchdringbar ist.

5. Blutentnahmevorrichtung nach einem der Ansprüche 2 bis 4, dadurch gekennzeichnet, daß der andere Stopfen (20) von Hand abziehbar ausgebildet ist.

6. Blutentnahmevorrichtung nach einem der Ansprüche 1 bis 2, durch gekennzeichnet, daß beide Röhrchen (12, 14) an einem Ende mittels eines von einer Kanüle durchdringbar ausgebildeten Stopfen (18) verschlossen sind, und eine aus dem anderen, offenen Ende des inneren Röhrchens (12) herausragende Kolbenstange (24) an das von dem inneren Röhrchen (12) aufgenommene Trennelement (10) über eine Sollbruchstelle (223) angesetzt ist.

7. Blutentnahmevorrichtung nach einem der vorangehenden Ansprüche, dadurch gekennzeichnet, daß die Länge des inneren-Röhrchens (12) geringer ist als die Länge des äußeren Röhrchens (14).

8. Blutentnahmevorrichtung nach einem der vorangehenden Ansprüche, dadurch gekennzeichnet, daß das äußere Röhrchen (14) an seinem offenen Ende mit einer Griffplatte (34) versehen ist.

9. Blutentnahmevorrichtung nach einem der Ansprüche, 1 bis 8, dadurch gekennzeichnet, daß der Innendurchmesser des äußeren Röhrchens (14) im Bereich des von der Kanüle durchdringbar ausgebildeten Stopfens (18) bzw. des Kanülenstutzens (26) dem Außendurchmesser des inneren Röhrchens (12) entsprechend ausgebildet ist.

## Claims

1. Blood sampling device with a first tube (12) made from a resilient material and which receives a separating element (10) and which expands during centrifuging, thus permitting an axial displacement of the separating element (10) otherwise engaging in sealing manner on the tube wall, characterized by a second tube (14) made from a not significantly resilient material, which surrounds the first tube (12), accompanied by the formation of an annular clearance (16) between the walls of the two tubes (12, 14) and means for sealing at least one end of the first tube.

2. Blood sampling device according to claim 1, characterized in that the two tubes (12, 14) are sealed at at least one of the two ends by means of a plug (18), which can be penetrated by a cannula.

3. Blood sampling device according to claim 2, characterized in that the two tubes (12, 14) are sealed at both ends by means of plugs (18, 20).

4. Blood sampling device according to claims 2 or 3, characterized in that one plug (18) can be penetrated by a cannula.

5. Blood sampling device according to one of the claims 2 to 4, characterized in that the other plug (20) can be drawn out manually.

6. Blood sampling device according to one of the claims 1 or 2, characterized in that the two tubes (12, 14) are sealed at one end by means of a plug (18), which can be penetrated by a cannula and a piston rod (24) projecting out of the other, open end of the inner tube (12) is attached by means of a predetermined breaking point (22) to the separating element (10) received by the inner tube (12).

7. Blood sampling device according to one of the preceding claims, characterized in that the inner tube (12) is shorter than the outer tube (14).

8. Blood sampling device according to one of the preceding claims, characterized in that the open end of the outer tube (14) is provided with a handle plate (34).

9. Blood sampling device according to one of the claims 1 to 8, characterized in that the internal diameter of the outer tube (14) in the vicinity of the cannula connection (26) or the plug (18) penetratable by the cannula, corresponds to the eternal diameter of the inner tube (12).

## Revendications

1. Appareil de prise de sang comportant un premier petit tube (12) en matière souple qui contient un élément séparateur (10) et, à la centrifugation, s'élargit et permet ainsi un déplacement axial de l'élément séparateur (10), autrement appliqué de manière étanche contre la paroi du petit tube, caractérisé par le fait qu'il contient un deuxième petit tube (14) en matière pratiquement rigide qui entoure le premier petit tube (12) en formant un interstice annulaire (16) entre les parois des deux petits tubes (12, 14), et des moyens de bouchage d'au moins une extrémité du premier petit tube.

2. Appareil de prise de sang selon la revendication 1, caractérisé par le fait que les deux petits tubes (12, 14) sont bouchés à au moins une des deux extrémités par un bouchon (18), un bouchon (18) pouvant être percé par une canule.

3. Appareil de prise de sang selon la revendication 2, caractérisé par le fait que les deux petits tubes (12, 14) sont bouchés aux deux extrémités par des bouchons (18, 20).

4. Appareil de prise de sang selon l'une des revendications 2 et 3, caractérisé par le fait qu'un bouchon (18) peut être percé par une canule.

5. Appareil de prise de sang selon l'une des revendications 2 à 4, caractérisé par le fait que l'autre bouchon (20) est fait de façon à pouvoir être retiré à la main.

6. Appareil de prise de sang selon l'une des revendications 1 et 2, caractérisé par le fait que les deux petits tubes (12, 14) sont bouchés à une extrémité par un bouchon (18) fait de façon à pouvoir être percé par une canule, et une tige de piston (24) saillant de l'autre extrémité, ouverte, du petit tube intérieur (12) est jointe par un point de rupture (22) à l'élément séparateur (10) placé dans le petit tube intérieur (12).

7. Appareil de prise de sang selon l'une des revendications précédentes, caractérisé par le fait que la longueur du petit tube intérieur (12) est inférieure à celle du petit tube extérieur (14).

8. Appareil de prise de sang selon l'une des revendications précédentes, caractérisé par le fait que le petit tube extérieur (14) est pourvu à son extrémité ouverte d'une plaque de préhension (34).

9. Appareil de prise de sang selon l'une des revendications 1 à 8, caractérisé par le fait que le diamètre intérieur du petit tube extérieur (14), dans la zone du bouchon (18) fait de façon à pouvoir être percé par la canule, ou de l'embout à canule (26), correspond au diamètre extérieur du petit tube intérieur (12).
